# EUROPEAN PATENT APPLICATION

(11) **EP 2 680 217 A1**
(43) Date of publication of application: **01.01.2014**
(21) Application number: 11859180.9
(22) Date of filing: 23.02.2011
(51) Int. Cl.: G06T 1/00

(54) **BIOLOGICAL INFORMATION ACQUISITION DEVICE AND BIOLOGICAL INFORMATION ACQUISITION METHOD**

(71) Applicant: Fujitsu Frontech Limited, Inagi-shi Tokyo 206-8555 (JP)
(72) Inventor: KAMATA, Hideo, Inagi-shi Tokyo 206-8555 (JP); MINAGAWA, Akitaka, Inagi-shi Tokyo 206-8555 (JP); HIGASHIURA, Yasuyuki, Inagi-shi Tokyo 206-8555 (JP); KASUGAI, Kentarou, Inagi-shi Tokyo 206-8555 (JP); IDE, Katsumi, Inagi-shi Tokyo 206-8555 (JP); TANAKA, Hiroyuki, Inagi-shi Tokyo 206-8555 (JP); YOSHIDA, Takashi, Inagi-shi Tokyo 206-8555 (JP)
(74) Representative: HOFFMANN EITLE
(86) International application number: PCT/JP2011/053979
(87) International publication number: WO 2012/114474

(57) **Abstract**

To adequately acquire biometric information even when a feature amount of the biometric information may vary by a variety of factors.

A biometric information acquisition apparatus (1) acquires biometric information to be used for verification. The biometric information acquisition apparatus (1) includes a blood flow increasing unit (2a), a biometric information acquiring unit (2b), a feature amount evaluating unit (2c), and a reacquisition determining unit (2d). The blood flow increasing unit (2a) increases blood flow of an object person. The biometric information acquiring unit (2b) acquires the biometric information from the object person. The feature amount evaluating unit (2c) evaluates the feature amount of the acquired biometric information. The reacquisition determining unit (2d) determines whether to cause the blood flow increasing unit (2a) to operate and then cause the biometric information acquiring unit (2b) to reacquire the biometric information when the evaluated feature amount does not reach a predetermined threshold.

## Description

### Technical Field

The embodiments discussed herein are related to biometric information acquisition apparatus and method for acquiring biometric information.

### Background Art

The human body has biometric information allowing an individual person to be positively identified. Some of this information is used to identify and authenticate individuals. Known biometrics for person authentication include those based on, for example, fingerprints, eye retina and iris patterns, facial characteristics, blood vessel patterns, and DNA (deoxyribonucleic acid).

Led by the development of biometric authentication technology in recent years, a great variety of apparatuses for recognizing body features of such human body parts and authenticating individuals have been offered. Biometric authentication is achieved by comparing biometric information collected upon registration (also referred to as a registered template) against biometric information newly acquired during the authentication.

In order to improve accuracy of biometric authentication using such biometric information, it is preferable to acquire biometric information with a certain degree of accuracy in each authentication attempt. However, a user which is an authentication object does not always assume a proper posture in the authentication process. In view of this, there is proposed a biometric information matching apparatus capable of evaluating a feature amount of biometric information and, then, prompting a user for re-entry to acquire biometric information of good quality if the feature amount is evaluated to be insufficient (see, for example, Patent Literature 1).

### Citation List

### Patent Literature

PTL1: Japanese Laid-open Patent Publication No. 2007-172022

### Summary of Invention

### Technical Problem

However, the feature amount included in the acquired biometric information varies among different individuals, and re-entry prompt does not always improve the feature amount immediately. In addition, reacquisition of biometric information does not lead to an improvement of the feature amount to be captured once a user has familiarized himself or herself, to a certain extent, with the procedure involved in the authentication process. Repeating re-entry prompt in spite of such a situation would deny a posture recognized by the user to be proper.

Especially, the aforementioned problem becomes much more pronounced in the case where the feature amount of biometric information to be acquired may vary by a variety of factors, such as a surrounding environment and a physical condition of the user.

In view of the above-described problem, the embodiments aim at providing biometric information acquisition apparatus and method capable of adequately acquiring biometric information even when the feature amount of the biometric information may vary by a variety of factors.

### Solution to Problem

In order to solve the above-described problem, there is provided a biometric information acquisition apparatus including a blood flow increasing unit, a biometric information acquiring unit, a feature amount evaluating unit, and a reacquisition determining unit. The blood flow increasing unit increases an amount of blood flow of an object person. The biometric information acquiring unit acquires, from the object person, biometric information whose feature amount is to be increased due to an increase in the amount of blood flow. The feature amount evaluating unit evaluates the feature amount of the biometric information acquired by the biometric information acquiring unit. The reacquisition determining unit determines whether to cause the blood flow increasing unit to operate and then cause the biometric information acquiring unit to reacquire the biometric information when the feature amount evaluated by the feature amount evaluating unit does not reach a predetermined threshold.

In order to solve the above-described problem, there is also provided a biometric information acquisition method including acquiring, from an object person, biometric information whose feature amount is to be increased due to an increase in an amount of blood flow of the object person; evaluating the feature amount of the acquired biometric information; and increasing the amount of blood flow in a biometric information acquisition region from which the biometric information of the object person is acquired and reacquiring the biometric information from the biometric information acquisition region when the evaluated feature amount does not reach a predetermined threshold.

### Advantageous Effects of Invention

The aforementioned biometric information acquisition apparatus and method enable adequate acquisition of biometric information even when the feature amount of the biometric information may vary by a variety of factors.

These and other objects, features and advantages of the present invention become apparent from the following detailed description of illustrative embodiments thereof, which is to be read in connection with the accompanying drawings.

### Brief Description of Drawings

[FIG. 1] FIG. 1 illustrates a configuration of a biometric information acquisition apparatus according to a first embodiment.
[FIG. 2] FIG. 2 illustrates a configuration of an authentication system according to a second embodiment.
[FIG. 3] FIG. 3 illustrates a configuration of a registration apparatus according to the second embodiment.
[FIG. 4] FIG. 4 illustrates a hardware configuration example of the registration apparatus according to the second embodiment.
[FIG. 5] FIG. 5 illustrates an overview of a sensor unit according to the second embodiment.
[FIG. 6] FIG. 6 illustrates a configuration of the sensor unit according to the second embodiment.
[FIG. 7] FIG. 7 illustrates observed examples of palmar surface temperature distribution according to the second embodiment.
[FIG. 8] FIG. 8 illustrates a flowchart of a biometric information acquiring process according to the second embodiment.
[FIG. 9] FIG. 9 illustrates a flowchart of a feature amount evaluating process according to the second embodiment.
[FIG. 10] FIG. 10 illustrates a flowchart of a blood flow increasing process according to the second embodiment.
[FIG. 11] FIG. 11 illustrates examples of vein images each having a different feature amount according to the second embodiment.
[FIG. 12] FIG. 12 illustrates examples of feature data sets extracted from the corresponding vein images according to the second embodiment.
[FIG. 13] FIG. 13 illustrates an example of feature amount evaluation results according to the second embodiment.
[FIG. 14] FIG. 14 illustrates examples of registered templates according to the second embodiment.
[FIG. 15] FIG. 15 illustrates an overview of an automated teller machine according to the second embodiment.
[FIG. 16] FIG. 16 illustrates a configuration of an authentication system according to a third embodiment.
[FIG. 17] FIG. 17 illustrates an overview of a sensor unit according to a fourth embodiment.
[FIG. 18] FIG. 18 illustrates an example of registered templates according to a fifth embodiment.

### Description of Embodiments

Several embodiments will be described below with reference to the accompanying drawings.

### First Embodiment

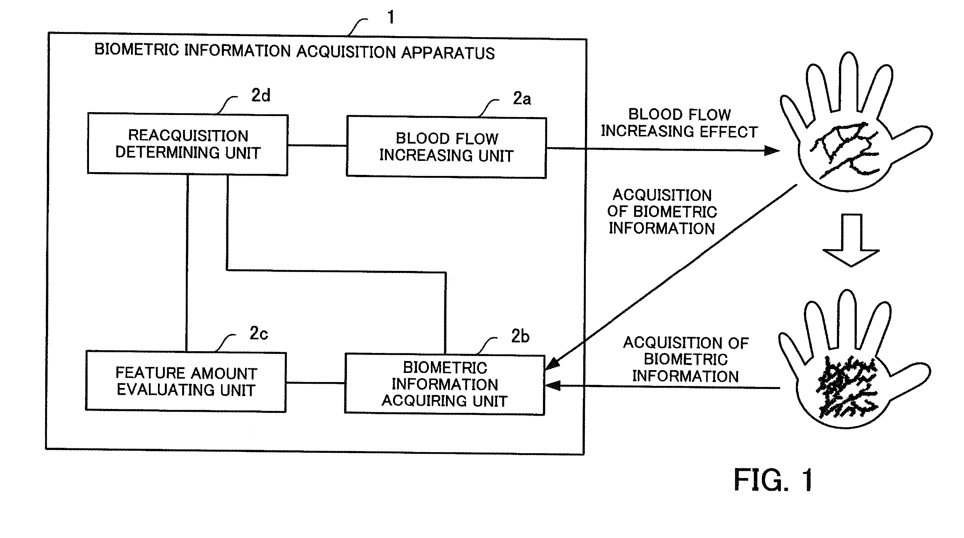

A biometric information acquisition apparatus according to a first embodiment is described first with reference to FIG. 1. FIG. 1 illustrates a configuration of a biometric information acquisition apparatus according to the first embodiment.

A biometric information acquisition apparatus 1 is configured to acquire, from a living body, biometric information whose feature amount (i.e., amount of feature information) is altered by a change in blood flow. Examples of such biometrics include those based on vein patterns near the body surface, such as a palm vein pattern and a finger vein pattern. In addition, the temperature of the body surface and the amount of perspiration are also examples of such biometric information.

The biometric information acquisition apparatus 1 may be configured as a stand-alone apparatus separate from an authentication apparatus for identity authentication using biometric information and a registration apparatus for registering biometric information (templates) used by the authentication apparatus for matching purposes, or may be configured as part of these apparatuses.

The biometric information acquisition apparatus 1 includes a blood flow increasing unit 2a, a biometric information acquiring unit 2b, a feature amount evaluating unit 2c, and a reacquisition determining unit 2d. The blood flow increasing unit 2a increases the blood flow of an object person. For example, in the case where biometric information to be acquired is a palm vein pattern, the blood flow increasing unit 2a warms a palm of the object person to increase blood flow. The biometric information acquiring unit 2b acquires, from the object person, biometric information whose feature amount is to be increased due to an increase in the blood flow. The feature amount evaluating unit 2c evaluates the feature amount of the biometric information acquired by the biometric information acquiring unit 2b. The reacquisition determining unit 2d determines whether to cause the blood flow increasing unit 2a to operate and then cause the biometric information acquiring unit 2b to reacquire the biometric information in the case where the feature amount evaluated by the feature amount evaluating unit 2c does not reach a predetermined threshold.

The term "feature amount" as used herein means the amount of significant information (feature information) allowing, among records of biometric information, identification determination (matching) of biometric information records extracted from the same person. In the case of, for example, a palm vein pattern, the feature information may be minutiae points in the vein pattern (ending points and bifurcation points of veins), the number of veins intersected by a straight line drawn between a minutiae point and an adjacent minutiae point, or a small partial image around a minutiae point.

The amount of such feature information varies from person to person, and some people present a high amount of feature information while others present a low amount of feature information. A person with a high amount of feature information tends to maintain high authentication accuracy, but a person with a low amount of feature information tends to be troubled by unstable authentication accuracy. In addition, the feature amount extracted from the same individual may be affected and vary by changes in the environment. The blood flow increasing unit 2a increases blood flow of the object person to thereby increase the amount of feature information to be acquired.

For example, a vein pattern acquired from a user (object person) with reduced blood flow caused by blood vessel constriction on a cold day includes a very low feature amount. In such a case, the blood flow increasing unit 2a warms a palm of the object person, for example, by a hot-air heater, which increases blood flow of the object person, in turn increasing the feature amount to be included in the vein pattern. The biometric information having the increased feature amount due to the increase in blood flow is acquired by the biometric information acquiring unit 2b from the object person.

As described above, even when the feature amount to be acquired may vary by a variety of factors, the biometric information acquisition apparatus 1 increases blood flow of an object person if biometric information including a very low feature amount has been acquired from the object person, to thereby acquire the biometric information with an increase feature amount. In addition, the biometric information acquisition apparatus 1 reduces incidence of repeatedly imposing re-entry of the biometric information on the user as a result of a verification failure, contributing to controlling a reduction in the level of convenience for the user. An authentication process using the biometric information acquired in this manner is expected to achieve stably high authentication accuracy.

Next, a detailed description is given using a second embodiment.

### Second Embodiment

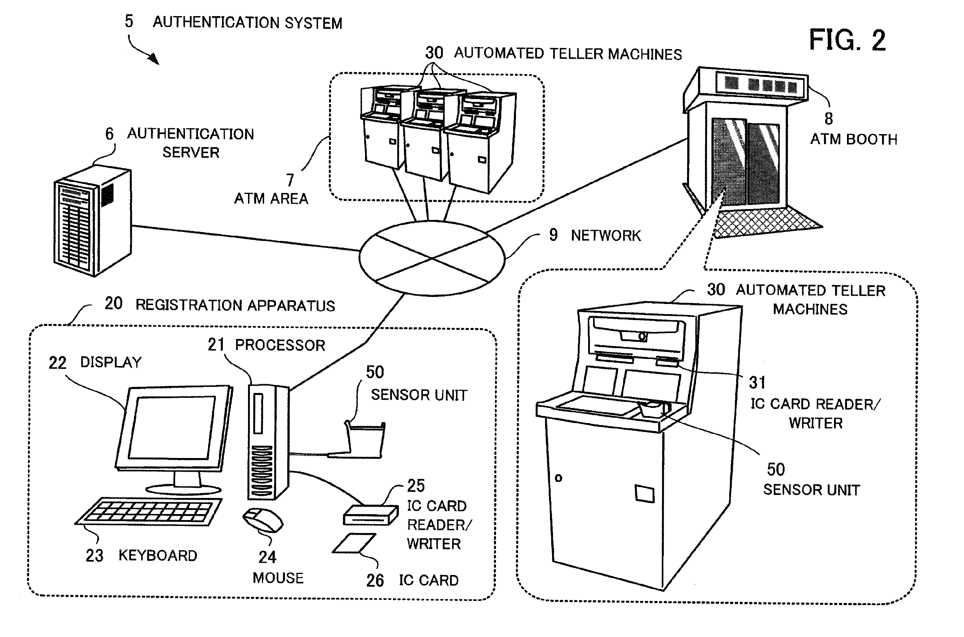

FIG. 2 illustrates a configuration of an authentication system according to a second embodiment. The second embodiment represents an authentication system 5 using veins in a palm for authentication, however, the embodiment is also applicable to a system utilizing a body feature other than palm veins for authentication as long as the feature amount of the body feature is altered by a change in the amount of blood flow.

The authentication system 5 is a type of system for recognizing features of a living body to thereby identify and authenticate an individual, and is used, for example, for client authentication in a banking system. The authentication system 5 includes a registration apparatus 20, multiple automated teller machines (ATM) 30, an authentication server 6, and a network 9.

The authentication server 6 stores, in association with each other, identification information for identifying each individual and verification information (template) registered in advance prior to biometric authentication. The identification information is a unique identification (ID) assigned directly (e.g., a user number) or indirectly (e.g., an account number) to a user. The verification information is, for example, feature information extracted from image information in relation to features by a predetermined feature extraction algorithm, or encoded information generated by encoding the image information or the feature information.

One or more automated teller machines 30 are installed in an ATM area 7 located inside a financial institution and an ATM booth 8. The automated teller machines 30 are authentication apparatuses used for biometric authentication to authenticate a user prior to a financial transaction. Each of the automated teller machines 30 includes an integrated circuit (IC) card reader/writer 31 and a sensor unit 50. The sensor unit 50 includes an image pickup device to take an image of palm veins of the user. The automated teller machine 30 authenticates the user based on verification information and biometric information of the user. The verification information is identified from identification information read by the IC card reader/writer 31 from an IC card (for example, a cash card having an IC chip mounted thereon) of the user. The biometric information of the user is acquired from the sensor unit 50. The sensor unit 50 is a biometric information acquisition apparatus for acquiring biometric information, and the automated teller machine 30 is an authentication apparatus including the biometric information acquisition apparatus.

The registration apparatus 20 is installed at a bank counter or the like, and used for registering a template of each user according to instructions or operations of a bank teller. The registration apparatus 20 includes a processor 21, a display 22, and the sensor unit 50, and may further include a keyboard 23, a mouse 24, and an IC card reader/writer 25 if needed. The sensor unit 50 includes a built-in image pickup device to capture an image of a palm of the user, which image is output to the processor 21. The IC card reader/writer 25 reads and writes information from and to an IC card 26 of the user. The keyboard 23 and the mouse 24 individually accept input operations.

Next described is template registration (registration of verification information) using the registration apparatus 20. A user calling for template registration enters identification information for identifying the user (for example, a user identification) using the keyboard 23, the mouse 24, or the IC card reader/writer 25. The registration apparatus 20 introduces the user to template registration procedures using a screen of the display 22 and prompts the user to enter biometric information for the template registration. The user enters biometric information by laying a hand over the sensor unit 50. The registration apparatus 20 into which an image of palm veins has been entered as biometric information creates verification information from the entered information and stores the verification information in at least one of a storage section of the processor 21, a storage section of the authentication server 6, and a storage section on the IC card 26 of the user. To carry out biometric authentication, the automated teller machine 30 makes an inquiry to the storage section of the authentication server 6 or the IC card 26 for a corresponding template and compares the entered biometric information against the template.

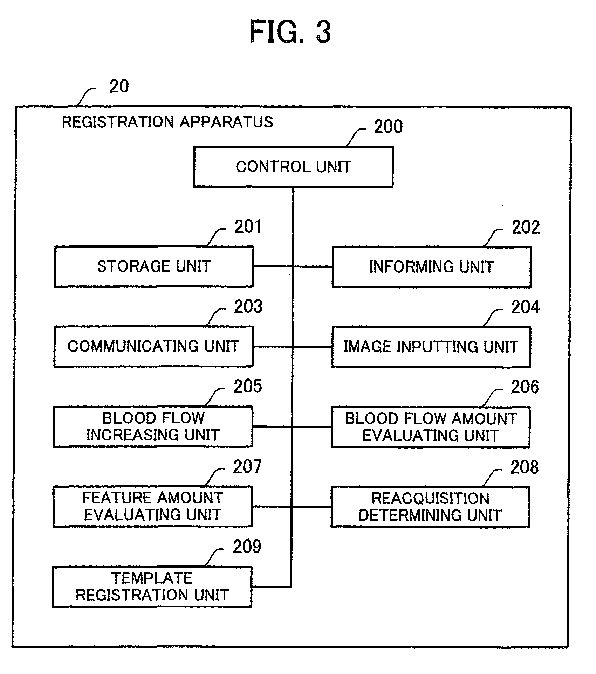

Next described is a configuration of the registration apparatus 20 of the second embodiment for achieving the template registration process, with reference to FIG. 3. FIG. 3 illustrates a configuration of a registration apparatus according to the second embodiment. The registration apparatus 20 includes a control unit 200, a storage unit 201, an informing unit 202, and a communicating unit 203. In addition, the registration apparatus 20 further includes an image inputting unit 204, a blood flow increasing unit 205, a blood flow amount evaluating unit 206, a feature amount evaluating unit 207, a reacquisition determining unit 208, and a template registration unit 209.

The control unit 200 exerts overall control over individual processing units to authenticate a user. The storage unit 201 stores and holds therein image information acquired from the sensor unit 50 and various databases. The informing unit 202 generates display messages needed, for example, to provide the user with guidance for the action of laying a hand over the sensor unit 50 and to inform the user of the temperature of the palm, the success or failure of the verification and the like, and then presents such a message on the display 22. The informing unit 202 also generates a needed audio message and audio-outputs the message using speakers (not illustrated). The communicating unit 203 communicates individually with the sensor unit 50, an IC chip built in the IC card reader/writer 25, and a computer connected to the network 9.

The image inputting unit 204 inputs a captured image of a living body part from the sensor unit 50. More specifically, upon inputting therein the captured image, the image inputting unit 204 extracts an object by deleting the background from the image and, then, determines whether the extracted object is a palm. If determining that the object is not a palm, the image inputting unit 204 inputs a captured image of a living body part once again from the sensor unit 50. On the other hand, if determining that the object is a palm, the image inputting unit 204 crops the palm (fingers and wrist may be included) from the captured image, and provides position correction of the cropped palm in terms of the position (position correction in the front-back and left-right directions), the size (height correction in the up-down direction), and the orientation (rotation correction).

The blood flow increasing unit 205 increases blood flow in a region from which biometric information is acquired (i.e., a palm of the user). For example, the blood flow increasing unit 205 includes a warming unit which warms the palm to thereby improve the flow of blood, in turn increasing blood flow. In addition, the blood flow increasing unit 205 includes a posture changing unit which changes the posture of the palm to thereby improve the flow of blood, in turn increasing blood flow. Further, the blood flow increasing unit 205 includes a vibrating unit which applies a stimulus to the palm to thereby improve the flow of blood, in turn increasing blood flow. The blood flow increasing unit 205 increases blood flow in the palm using one of the warming unit, the posture changing unit, and the vibrating unit, or any combination of these units. Note that the blood flow increasing unit 205 may be configured to trigger the user's vision, hearing or other senses rather than the above-mentioned somatic senses as long as the sense has a causal connection with an increase in blood flow. In this case, the blood flow increasing unit 205 increases blood flow, for example, by presenting a predetermined image on the display 22 to stimulate vision of the user or by outputting predetermined audio content from speakers to stimulate his or her hearing. Because a blood flow increasing effect is generally a vasodilatory effect, the blood flow increasing unit 205 may stimulate the user's parasympathetic nervous system that has a vasodilatory effect.

The blood flow amount evaluating unit 206 evaluates the amount of blood flow in the region from which biometric information is acquired (i.e., a palm of the user). For example, the blood flow amount evaluating unit 206 includes a temperature measuring unit which evaluates the amount of blood flow based on the temperature of the palm. More specifically, the blood flow amount evaluating unit 206 detects a rise in body temperature, based on which an increase in blood flow in the region for the biometric information acquisition is evaluated.

The feature amount evaluating unit 207 evaluates a feature amount included in the biometric information based on the captured image. More specifically, the feature amount evaluating unit 207 evaluates a vein pattern in the palm image or the amount of feature information included in the vein pattern. The feature information may be minutiae points in the vein pattern (ending points and bifurcation points of veins), the number of veins intersected by a line drawn between a minutiae point and an adjacent minutiae point, or a small partial image around a minutiae point. The amount of feature information is a quantified representation of the number or quality of the feature information obtained by a predetermined evaluation algorithm. A higher amount of feature information results in authentication with stable accuracy while a lower amount of feature information leads to authentication with less stable accuracy. A simplified example of the amount of feature information is the sum of the count of minutiae points and the number of veins intersected by a line drawn between a minutiae point and an adjacent minutiae point. Note that the evaluation of the feature amount by the feature amount evaluating unit 207 may be represented by an evaluation value (for example, a numerical value such as 100 and 200), or a score on an evaluation scale denoting the quantity of the feature amount based on predetermined thresholds (for example, a score on a scale of "high", "moderate", and "low").

The reacquisition determining unit 208 determines need of reacquiring the biometric information based on the evaluation of the feature amount. If the feature amount of the acquired biometric information is evaluated to be insufficient, the reacquisition determining unit 208 determines reacquisition of the biometric information, that is, re-entry of an image.

The template registration unit 209 processes the extracted image information to be a registration template, and records (registers) the registration template in the storage section of the processor 21, the storage section of the authentication server 6, or the storage section on the IC card 26 of the user.

Note that the registration apparatus 20 increases blood flow in the region for the biometric information acquisition for the purpose of increasing the feature amount included in the biometric information of the template registration target. On the other hand, an authentication apparatus does the same for the purpose of increasing the feature amount included in biometric information of a verification target. Therefore, the authentication apparatus may have the same configuration as the registration apparatus, except for the template registration unit 209 which is replaced with a verifying unit. The verifying unit carries out biometric verification by comparing biometric information extracted from a captured image input by the image inputting unit 204 against a registration template registered in advance.

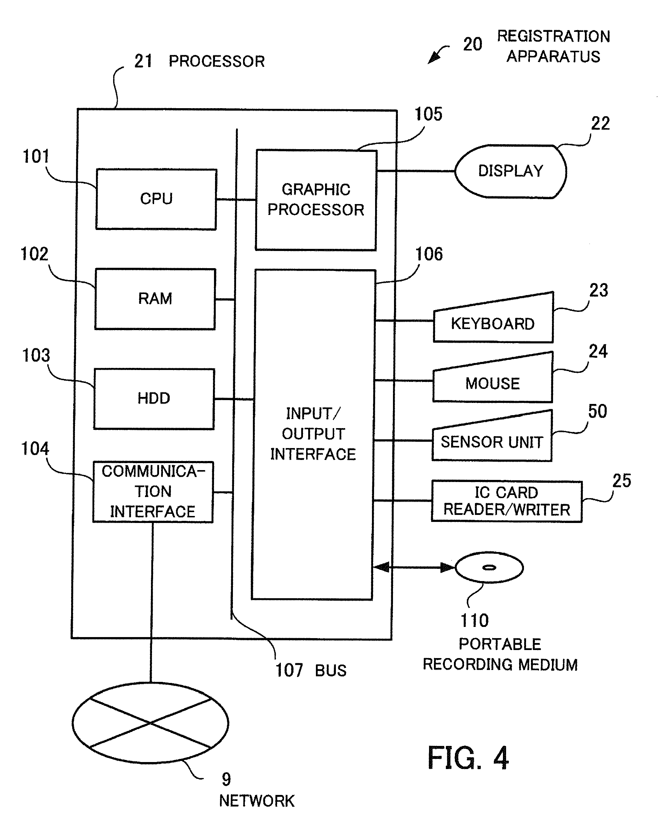

Next described is a hardware configuration example of the registration apparatus 20 according to the second embodiment, with reference to FIG. 4. FIG. 4 illustrates a hardware configuration example of a registration apparatus according to the second embodiment.

The registration apparatus 20 includes the processor 21, the display 22, the keyboard 23, the mouse 24, the sensor unit 50, and the IC card reader/writer 25.

The whole processor 21 is controlled by a central processing unit (CPU) 101. To the CPU 101, the following devices are connected via a bus 107: a random access memory (RAM) 102; a hard disk drive (HDD) 103; a communication interface 104; a graphic processor 105; and an input/output interface 106.

The RAM 102 temporarily stores therein at least part of an operating system program and application programs to be executed by the CPU 101. The RAM 102 also stores various types of data needed by the CPU 101 for its processing. The HDD 103 stores the operating system program and the application programs.

To the graphic processor 105, the display 22 is connected. The graphic processor 105 causes the display 22 to present an image on its screen according to an instruction of the CPU 101.

To the input/output interface 106, the keyboard 23, the mouse 24, the sensor unit 50, and the IC card reader/write 25 are connected. In addition, the input/output interface 106 is configured to be connected to a portable recording medium interface allowing information to be written and read to and from a portable recording medium 110. The input/output interface 106 transmits signals individually sent from the keyboard 23, the mouse 24, the sensor unit 50, the IC card reader/writer 25, the portable recording medium interface to the CPU 101 via the bus 107.

The communication interface 104 is connected to the network 9. The communication interface 104 transmits and receives data to and from another computer (for example, the authentication server 6).

The hardware configuration described above achieves the processing functions of this embodiment. Note that the authentication server 6 and the automated teller machine 30 may individually have the same hardware configuration.

Note that the processor 21 may include modules each composed of a field programmable gate array (FPGA), a digital signal processer (DSP) or the like, and may not include the CPU 101. In such a case, the processor 21 includes a nonvolatile memory (for example, an electrically erasable and programmable read-only memory (EEPROM), a flash memory, or a flash memory card), in which firmware of the modules is stored. The firmware may be written to the nonvolatile memory via the portable recording medium 110 or the communication interface 104. Thus, the processor 21 is able to update firmware by rewriting the firmware stored in the nonvolatile memory.

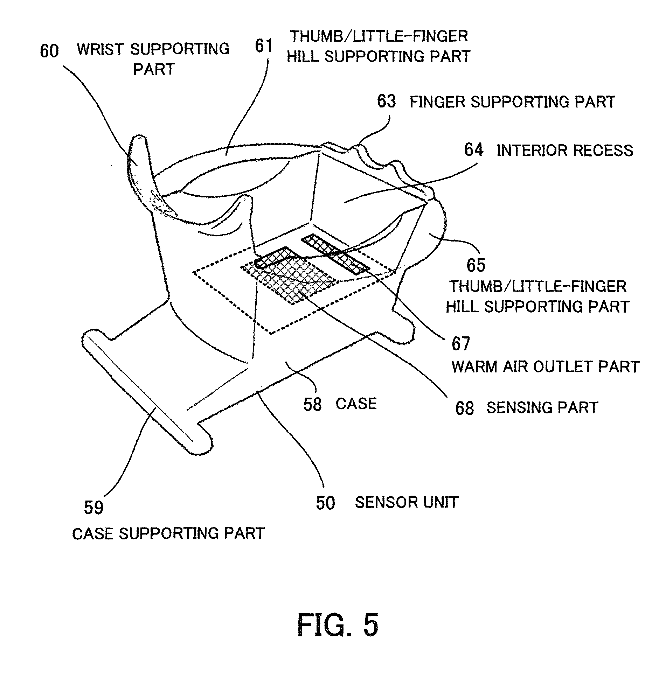

Next is described an overview of the sensor unit 50 according to the second embodiment, with reference to FIG. 5. FIG. 5 illustrates an overview of a sensor unit according to the second embodiment. The sensor unit 50 has a form in which a boxy case 58 with an open top is supported by a case supporting part 59 functioning as a base. The case 58 includes a wrist supporting part 60 for supporting a wrist of the user, a finger supporting part 63 for supporting fingers, and thumb/little-finger hill supporting parts 61 and 65 for supporting a thumb hill or a little finger hill. The wrist supporting part 60, the finger supporting part 63, the thumb/little-finger hill supporting parts 61 and 65 form four sides of the open top of the case 58 to provide good support for a palm of the user. In addition, the wrist supporting part 60 has a substantially U-shape configuration to support the wrist of the user in a correct posture. The finger supporting part 63 has a wave shape with two crests to support the index, third, and fourth fingers individually in their correct positions. Each of the thumb/little-finger hill supporting parts 61 and 65 has a concave shape with a curved surface to support the thumb hill or the little finger hill. With these supporting parts, the sensor unit 50 supports the palm in a correct position.

The boxy case 58 has a sensing part 68 and a warm air outlet part 67 on its bottom surface. The sensing part 68 includes a first image sensor (for example, a complementary metal-oxide semiconductor (CMOS) sensor, or a charge-coupled device (CCD) sensor) for capturing an image of a living body part, a condenser lens, multiple near-infrared light emitting devices (light emitting diodes (LED)) irradiating an object, and a second image sensor for capturing an image of temperature distribution of the living body part. The near-infrared light emitting devices emit near-infrared light in the direction of the object (i.e., the upper direction).

The warm air outlet part 67 includes a heater and a fan and blows out warm air toward the open top. As the heater, a nichrome wire coil, for example, is used. Alternatively, heaters may be provided in the parts with which the hand comes in contact, that is, the wrist supporting part 60, the finger supporting part 63, and the thumb/little-finger hill supporting parts 61 and 65, to thereby warm the contact areas of the hand. Further, a heater may be provided on the internal wall surface forming an interior recess 64 to warm the inside of the interior recess. The internal warm air is prevented by the interior recess 64 from diffusing and efficiently warms the palm of the user.

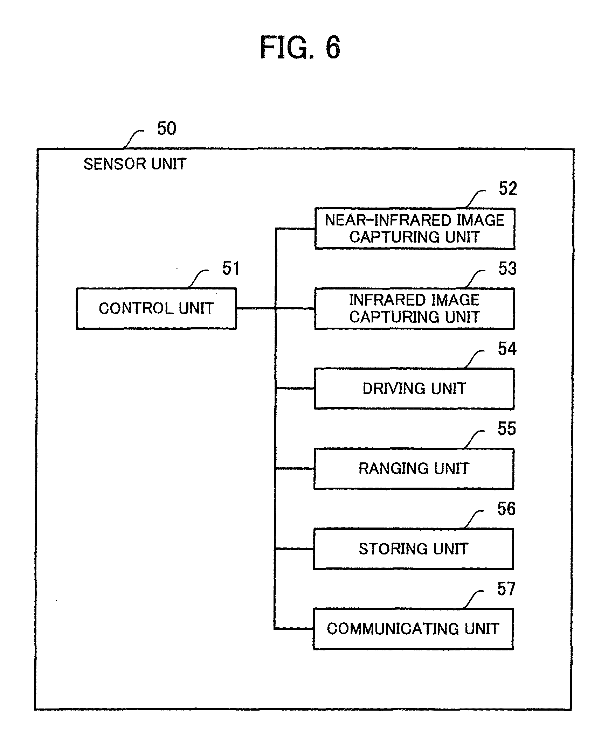

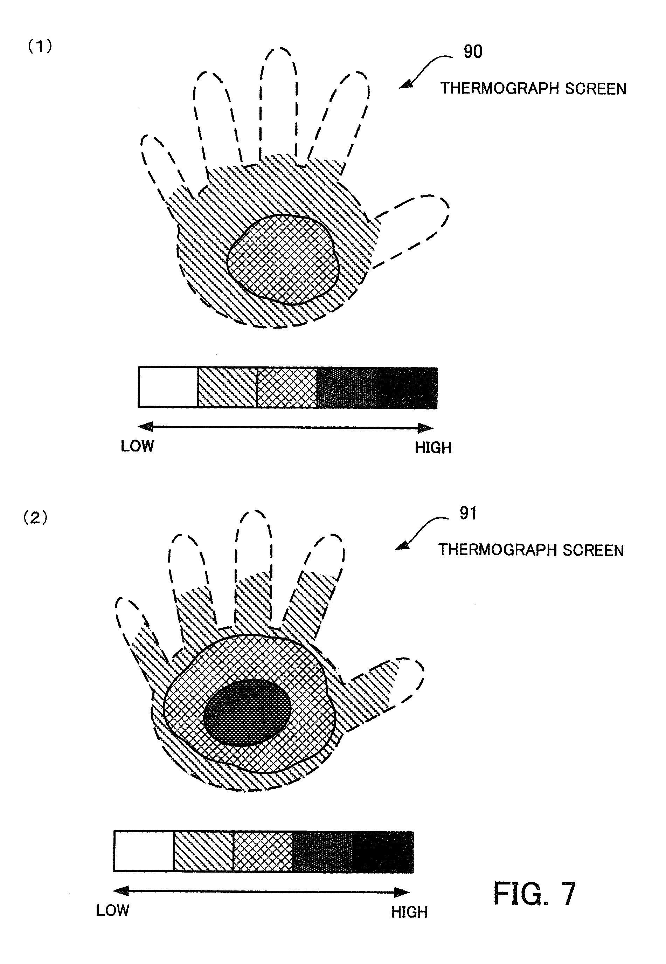

Next described are functions provided for the sensor unit 50 according to the second embodiment, with reference to FIGS. 6 and 7. FIG. 6 illustrates a configuration of a sensor unit according to the second embodiment. FIG. 7 illustrates observed examples of palmar surface temperature distribution according to the second embodiment.

The sensor unit 50 includes a control unit 51, a near-infrared image capturing unit 52, an infrared image capturing unit 53, a driving unit 54, a ranging unit 55, a storing unit 56, and a communicating unit 57.

The control unit 51 exerts overall control over individual processing units. The near-infrared image capturing unit (the first image sensor) 52 acquires image information from an object, that is a targeted living body part. The near-infrared image capturing unit 52 is able to continuously shoot the object, achieving continuous shooting of, for example, 15 frames per second. Note that the setting of the shooting speed may be changed. Alternatively, the shooting timing may be determined not according to time, but according to distance from the object obtained based on an output of the ranging unit 55. Note that the near-infrared image capturing unit 52 is configured suitable to capture an image of palm veins, and in the case of capturing an image of a tip of a finger or a different living body part, a configuration suitable for the object may be adopted.

The near-infrared image capturing unit 52 captures near-infrared light reflected from the object, i.e., the living body part (palm), to form an image. Since hemoglobin in red blood cells flowing through veins has lost oxygen, the hemoglobin (reduced hemoglobin) has the property of absorbing light in a near-infrared range of around 700 nm to around 1000 nm. Therefore, when near-infrared light hits a palm, less reflection is observed in areas where veins are located, and thus, it is possible to recognize the location of veins based on the intensity of the reflected near-infrared light. Images captured by the near-infrared image capturing unit 52 are achromatic, and the use of a specific light sauce facilitates extraction of characteristic information.

The infrared image capturing unit 53 captures an image of temperature distribution of the palm at an infrared wavelength (in general, in the range of 7.5 µm to 13 µm). Therefore, the infrared image capturing unit 53 has a function of measuring the temperature of the region from which biometric information is acquired. The temperature distribution of a palm captured in the image is not only used to evaluate the amount of blood flow, but also presented on the display 22. For example, the display 22 presents temperature distribution of the palm using thermograph screens starting with a thermograph screen 90 (refer to FIG. 7(1)) and ending with a thermograph screen 91 (FIG. 7(2)). Such temperature distribution enables understanding of whether the temperature of the palm is sufficient or not. In addition, the temperature transition enables understanding of whether the temperature of the palm has been increased and, correspondingly, blood flow has been increased.

The driving unit 54 powers up the heater and drives the fan. That is, the driving unit 54 functions as a warming unit to warm a palm by delivering warm air to the palm.

The ranging unit 55 measures the distance from a living body part being the object. The measured distance is used to determine shooting timing. The storing unit 56 stores therein image information acquired by the near-infrared image capturing unit 52. The communication unit 57 is connected to the communicating unit 203 of the registration apparatus 20 to thereby receive an instruction from the registration apparatus 20 and transmit image information and the like to the registration apparatus 20.

In the above-described manner, the sensor unit 50 warms a region from which biometric information is acquired, i.e., a palm of a user, to cause palm veins to dilate and increase blood flow. As a result, the near-infrared image capturing unit 52 captures an image of veins with the increased blood flow and, subsequently, the sensor unit 50 outputs a captured image with an increased feature amount.

In addition, the sensor unit 50 being provided with the infrared image capturing unit 53 allows the registration apparatus 20 to present temperature distribution of the palm on the display 22. The display of transition of the palm temperature distribution gives a justified reason to the user for waiting until the temperature of the palm has increased. Displaying model palm temperature distribution together with the palm temperature distribution of the user is likely to further reduce the user's frustration with the waiting time.

Note that the degree of warmth of a palm, that is, the degree of the increase in blood flow may be determined to be sufficient if the temperature distribution satisfies a predetermined criterion (for example, 36 degrees Celsius or more over 80% or more of the surface area of the palm). In addition, the predetermined criterion may be a temperature rise from the start of the warming up, a temperature rise range, the rate of the temperature rise, or some combination of the above.

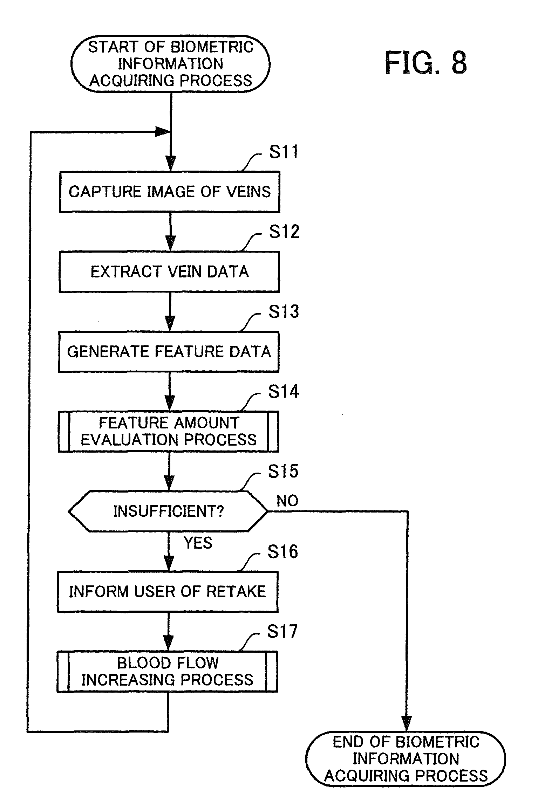

A biometric information acquiring process implemented by the processor 21 according to the second embodiment is next described in detail with reference to FIG. 8. FIG. 8 illustrates a flowchart of a biometric information acquiring process according to the second embodiment. In template registration, the processor 21 acquires biometric information to be registered as a template. At this point, the processor 21 implements the biometric information acquiring process to thereby acquire biometric information.

[Step S11] The processor 21 (the image inputting unit 204) makes a request to the sensor unit 50 for an image, in which palm veins are captured (captured image), to be used for template registration. The sensor unit 50 responds to the processor 21 with a captured image of a palm. The processor 21 acquires the captured image from the sensor unit 50.

[Step S12] The processor 21 (the feature amount evaluating unit 207) extracts vein data from the captured image by predetermined image processing.

[Step S13] The processor 21 (the feature amount evaluating unit 207) generates feature data from the extracted vein data.

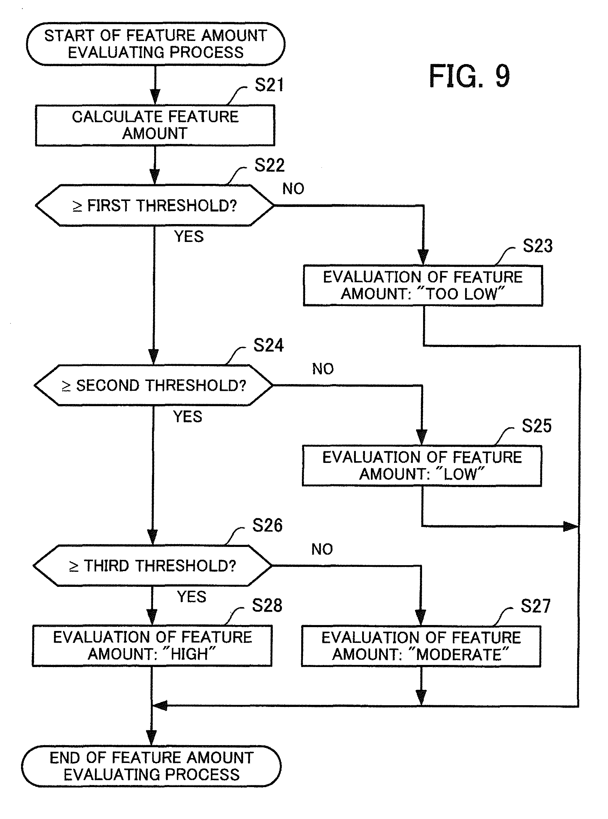

[Step S14] The processor 21 (the feature amount evaluating unit 207) implements a feature amount evaluating process for evaluating a feature amount included in the generated feature data. The details of the feature amount evaluating process are described later with reference to FIG. 9.

[Step S15] The processor 21 (the reacquisition determining unit 208) determines whether the feature amount included in the feature data is sufficient. The determination is made by comparing the feature amount with one or more predetermined thresholds. The processor 21 (the reacquisition determining unit 208) proceeds to step S16 if the feature amount is insufficient, and ends the biometric information acquiring process if the feature amount is sufficient.

[Step S16] The processor 21 (the informing unit 202) informs a user of a retake of a palm vein image. The processor 21 (the informing unit 202) also informs the user of a palm warming treatment to be provided. Further, the processor 21 (the informing unit 202) informs the user of the current status of the palm temperature distribution.

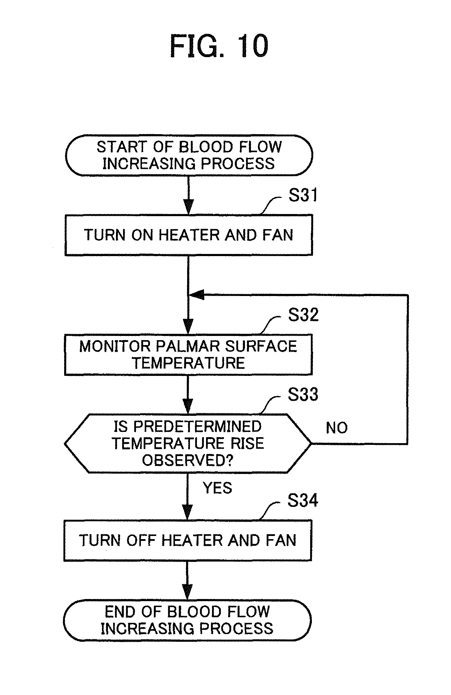

[Step S17] The processor 21 (the blood flow increasing unit 205) implements a blood flow increasing process for increasing blood flow in the palm (the region from which biometric information is acquired) and, subsequently, returns to step S11 after the end of the process implementation in order to take a palm vein image once again. The details of the blood flow increasing process are described later with reference to FIG. 10.

In this manner, the registration apparatus 20 acquires biometric information having a predetermined feature amount, which improves quality of the registration template and contributes to an improvement in authentication accuracy. Although the above description is directed to the case where, in template registration, the registration apparatus 20 acquires biometric information to be registered as a template, the same process may be used by an authentication apparatus (for example, the automated teller machine 30) to acquire biometric information in each authentication attempt.

The feature amount evaluating process implemented by the feature amount evaluating unit 207 according to the second embodiment is next described in detail with reference to FIG. 9. FIG. 9 illustrates a flowchart of a feature amount evaluating process according to the second embodiment. The feature amount evaluating process is carried out in step S14 of the biometric information acquiring process.

[Step S21] The feature amount evaluating unit 207 obtains a feature amount included in the feature data by quantifying the number or quality of feature information using a predetermined evaluation algorithm.

[Step S22] The feature amount evaluating unit 207 determines whether the calculated feature amount is equal to or more than a first threshold. The feature amount evaluating unit 207 proceeds to step S24 if the feature amount is equal to or more than the first threshold, and proceeds to step S23 if not.

[Step S23] The feature amount evaluating unit 207 evaluates that the calculated feature amount is "too low". Since the biometric information whose feature amount is evaluated as being "too low" is not able to guarantee a predetermined authentication accuracy, the feature amount is determined to be insufficient in step S15 of the biometric information acquiring process.

[Step S24] The feature amount evaluating unit 207 determines whether the calculated feature amount is equal to or more than a second threshold. The feature amount evaluating unit 207 proceeds to step S26 if the feature amount is equal to or more than the second threshold, and proceeds to step S25 if not.

[Step S25] The feature amount evaluating unit 207 evaluates that the calculated feature amount is "low". Since the biometric information whose feature amount is evaluated as being "low" may not be able to guarantee the predetermined authentication accuracy, the feature amount is determined to be insufficient in step S15 of the biometric information acquiring process. Note that the biometric information whose feature amount is evaluated as being "low" may be accepted from the aspect of convenience after no improvement is observed in reacquisition of biometric information. That is, even when determined to be insufficient once in step S15 of the biometric information acquiring process, the calculated feature amount is not determined to be insufficient in succession.

[Step S26] The feature mount evaluating unit 207 determines whether the calculated feature amount is equal to or more than a third threshold. The feature amount evaluating unit 207 proceeds to step S28 if the feature amount is equal to or more than the third threshold, and proceeds to step S27 if not.

[Step S27] The feature amount evaluating unit 207 evaluates that the calculated feature amount is "moderate" and, then, ends the feature amount evaluating process. Since the biometric information whose feature amount is evaluated as being "moderate" is able to guarantee the predetermined authentication accuracy, the feature amount is determined to be not insufficient in step S15 of the biometric information acquiring process.

[Step S28] The feature amount evaluating unit 207 evaluates that the calculated feature amount is "high" and, then, ends the feature amount evaluating process. Since the biometric information whose feature amount is evaluated as being "high" is able to sufficiently guarantee the predetermined authentication accuracy, the feature amount is determined to be not insufficient in step S15 of the biometric information acquiring process.

Thus, in the feature amount evaluating process, a feature amount is evaluated in multiple steps according to the magnitude of the feature amount. Note that the feature amount may be evaluated using a two-point scale of "suitable (high)" and "unsuitable (low)", a three-point scale of "optimal (high)", "suitable (moderate)", and "unsuitable (low)", or a scale with still more points.

The blood flow increasing process implemented by the blood flow increasing unit 205 according to the second embodiment is next described in detail with reference to FIG. 10. FIG. 10 illustrates a flowchart of a blood flow increasing process according to the second embodiment. The blood flow increasing process is carried out in step S17 of the biometric information acquiring process.

[Step S31] The blood flow increasing unit 205 turns on the heater and fan of the warm air outlet part 67.

[Step S32] The blood flow increasing unit 205 monitors the palmar surface temperature based on the palm temperature distribution obtained by the infrared image capturing unit 53.

[Step S33] The blood flow increasing unit 205 determines whether a temperature rise with the temperature distribution satisfying a predetermined criterion (for example, 36 degrees Celsius or more over 80% or more of the surface area of the palm) is observed. The blood flow increasing unit 205 proceeds to step S34 if a temperature rise satisfying the predetermined criterion is observed, and returns to step S32 if not and continues monitoring the palmar surface temperature.

[Step S34] The blood flow increasing unit 205 turns off the heater and fan of the warm air outlet part 67 and ends the blood flow increasing process.

In this manner, the blood flow increasing unit 205 warms a palm to increase the temperature of the palm, improving the flow of blood in the palm, that is, increasing blood flow in palm veins.

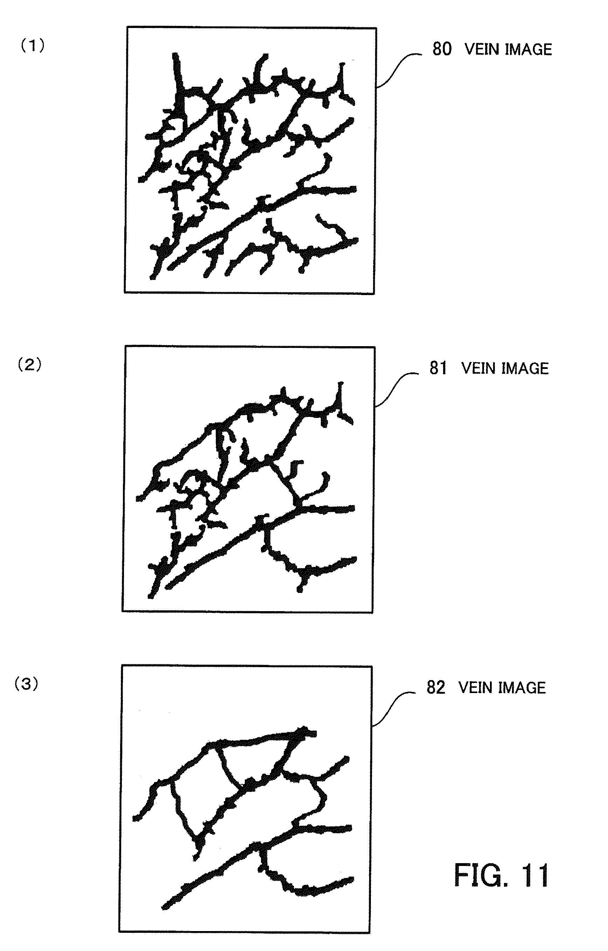

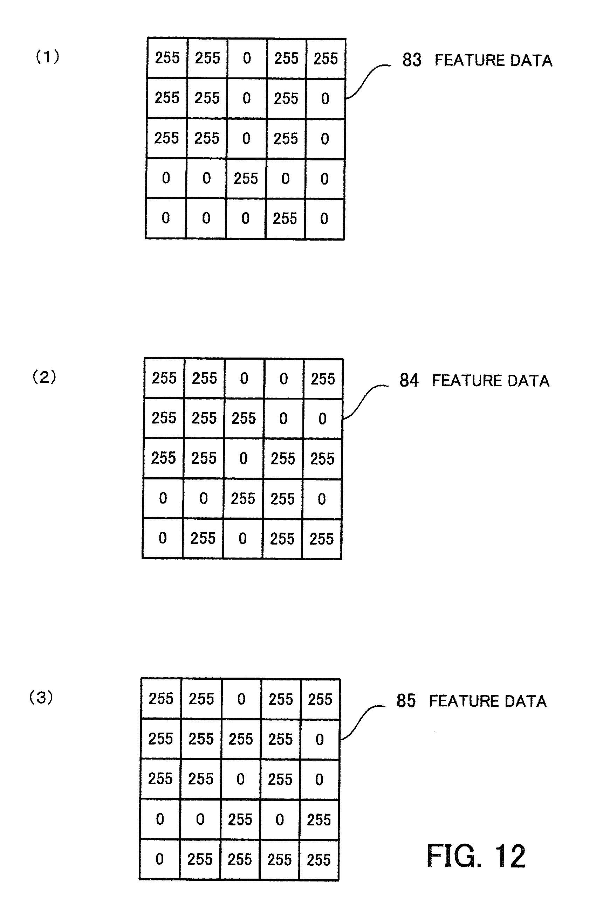

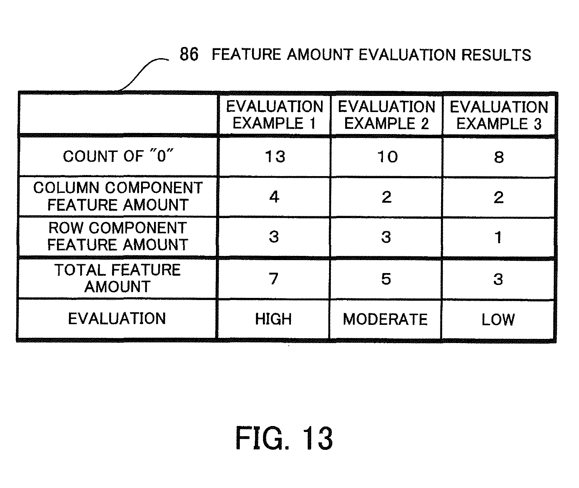

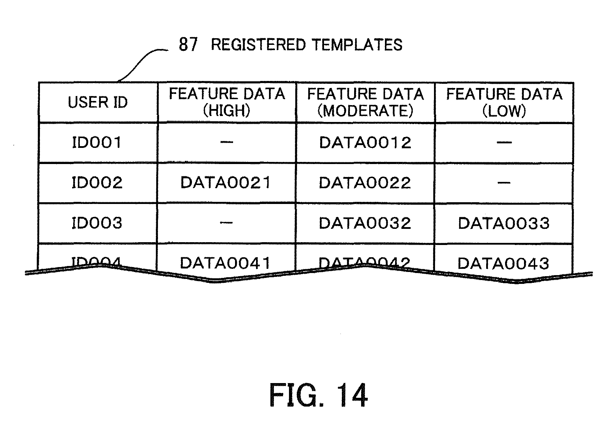

Next described are examples of acquired palm vein images and registered templates generated based on the palm vein images according to the second embodiment, with reference to FIGS. 11 to 14. FIG. 11 illustrates examples of vein images each having a different feature amount according to the second embodiment. FIG. 12 illustrates examples of feature data sets extracted from the corresponding vein images according to the second embodiment. FIG. 13 illustrates an example of feature amount evaluation results according to the second embodiment. FIG. 14 illustrates examples of registered templates according to the second embodiment.

The feature amount of a palm vein image (vein pattern) varies among different individuals and also varies according to the environment. A vein image 80 (see FIG. 11(1)) exhibits a relatively complex vein pattern including many bifurcation points of veins and having a large amount of veins per unit area. Such a vein image 80 is evaluated as having a high feature amount. A vein image 81 (see FIG. 11(2)) exhibits a vein pattern with average complexity, including a moderate number of bifurcation points of veins and having a moderate amount of veins per unit area. Such a vein image 81 is evaluated as having a moderate feature amount. A vein image 82 (see FIG. 11(3)) exhibits a relatively simple vein pattern including a small number of bifurcation points of veins and having a small amount of veins per unit area. Such a vein image 82 is evaluated as having a low feature amount.

The vein image 80 evaluated as having a high feature amount is said to be less subject to environmental influences, providing sufficient authentication accuracy even under environmental changes such as seasonal variation in temperature and variation in the ambient temperature. In addition, such a vein image 80 is also said to be less subject to variation in the posture of the user, providing sufficient authentication accuracy even when there are postural changes such as a standing or seated position, a position of the arm, and a wrist angle relative to the heart.

On the other hand, the vein image 82 evaluated as having a low feature amount is said to be susceptible to environmental influences, providing poor authentication accuracy under subtle environmental changes. In addition, such a vein image 82 is also said to be susceptible to variation in the posture of the user, providing poor authentication accuracy if there is a slight change in the posture.

The blood flow increasing unit 205 improves the vein image 82 evaluated as having a low feature amount to be one like the vein image 81 evaluated as having a moderate feature amount or the vein image 80 evaluated as having a high feature amount.

Each of the vein images 80, 81, and 82 is, for example, processed to generate a feature data set by binarizing corresponding image information with a 256-level gray scale, ranging from 0 to 255, into 0 and 255. A feature data set 83 (see FIG. 12(1)) is an example of feature data generated for a part of the vein image 80. A feature data set 84 (see FIG. 12(2)) is an example of feature data generated for a part of the vein image 81. A feature data set 85 (see FIG. 12(3)) is an example of feature data generated for a part of the vein image 82. The feature data sets 83, 84, and 85 are individually evaluated in terms of the feature amount, which results are presented as feature amount evaluation results 86 (see FIG. 13). The feature amount evaluation results 86 include the following entry items: the count of "0" included in each feature data set; a column component feature amount which is the count of sections with successive 0s in the column direction; a row component feature amount which is the count of sections with two or more successive 0s in the row direction; and a total feature amount which is the sum of the column and row component feature amounts. In addition, the feature amount evaluation results 86 also include an entry item of evaluation which is a categorized result obtained by classifying a corresponding total feature amount into one of categories of "low", "moderate", and "high" based on predetermined thresholds (see the first to third thresholds in FIG. 9).

According to the feature amount evaluation results 86 of FIG. 13, as for evaluation example 1 representing an evaluation result of the feature data set 83, the count of "0" is "13", the column component feature amount is "4", the row component feature amount is "3", and the total feature amount is "7". Assuming here that the total feature amount "7" is equal to or more than the third threshold, the feature data set 83 is evaluated as having a "high" feature amount. As for evaluation example 2 representing an evaluation result of the feature data set 84, the count of "0" is "10", the column component feature amount is "2", the row component feature amount is "3", and the total feature amount is "5". Assuming here that the total feature amount "5" is equal to or more than the second threshold but less than the third threshold, the feature data set 84 is evaluated as having a "moderate" feature amount. As for evaluation example 3 representing an evaluation result of the feature data set 85, the count of "0" is "8", the column component feature amount is "2", the row component feature amount is "1", and the total feature amount is "3". Assuming here that the total feature amount "3" is equal to or more than the first threshold but less than the second threshold, the feature data set 85 is evaluated as having a "low" feature amount.

Each of the feature data sets evaluated in this manner is associated with both a user identification for uniquely identifying a user and a feature amount evaluation, and registered as a template as illustrated in registered templates 87 (see FIG. 14). For example, a user with a user identification ID001 is associated with an evaluation result "moderate" and a feature data set DATA0012.

In addition, not only one but also multiple feature data sets may be associated with a single user identification. For example, a user with a user identification ID002 is associated with an evaluation result "high" and a feature data set DATA0021 as well as with an evaluation result "moderate" and a feature data set DATA0022.

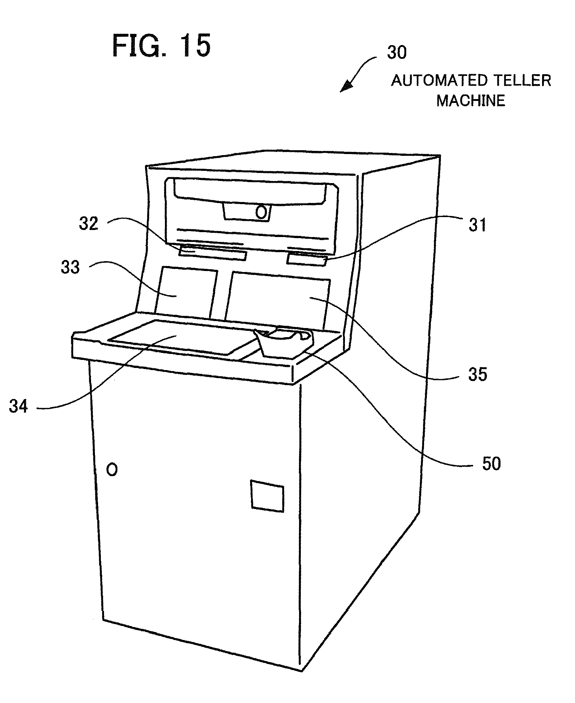

Thus, in the case where multiple feature data sets each having a different evaluation result have been registered as templates, a template (feature data set) for verification may be selected according to surrounding environmental conditions causing changes in the amount of blood flow, such as seasons and temperature variations. Next is described an overview of the automated teller machine 30 according to the second embodiment, with reference to FIG. 15. FIG. 15 illustrates an overview of an automated teller machine according to the second embodiment.

The automated teller machine 30 includes the IC card reader/writer 31, a bankbook insertion port 32, a coin deposit and withdrawal port 33, a display and operation unit 34, a banknote deposit and withdrawal port 35, and the sensor unit 50. The IC card reader/writer 31 receives a cache card (IC card) and reads and writes information stored in the IC card. The bankbook insertion port 32 receives a bank book. The coin deposit and withdrawal port 33 is a deposit and withdrawal port for coins. The banknote deposit and withdrawal port 35 is a deposit and withdrawal port for banknotes. The display and operation unit 34 is an image display device with a touch panel function, serving as a display output unit as well as an operation input unit. The sensor unit 50 has a function as a biometric information acquiring unit for capturing biometric information of a user as well as a function as a blood flow increasing unit for increasing blood flow of the user.

The automated teller machine 30 is installed in the ATM area 7 or the ATM booth 8, and in many cases, a user visits the ATM area 7 or the ATM booth 8 from outdoors. Therefore, a palm of the user having been exposed to the outside air, for example, on a cold day may be experiencing bad blood circulation. Even in such a case, the automated teller machine 30 ensures stable authentication using good biometric information having an increased feature amount because the biometric information of the user is captured after his or her blood flow has been increased. Such an automated teller machine 30 contributes to improving convenience for users and, also, offers improved operational efficiency by preventing repeated authentication failures. In addition, the automated teller machine 30 reduces the waiting time of the users.

### Third Embodiment

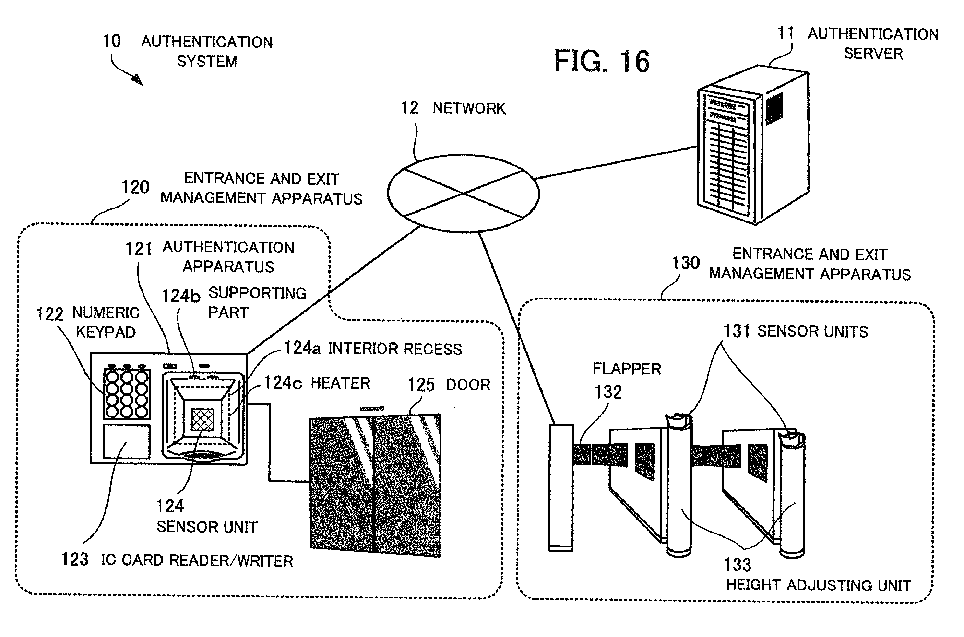

Next described is an authentication system according to a third embodiment. The authentication system according to the third embodiment is used to manage entrance and exit to and from a room. FIG. 16 illustrates a configuration of an authentication system according to the third embodiment. The third embodiment represents an authentication system 10 using veins in a palm for authentication, however, the embodiment is also applicable to a system utilizing a body feature other than palm veins for authentication as long as the feature amount of the body feature is altered by a change in the amount of blood flow.

The authentication system 10 is a system configured to recognize features of a living body to thereby identify and authenticate an individual, and is used, for example, in an entrance and exit management system to authenticate individuals entering and leaving a room. The authentication system 10 includes entrance and exit management apparatuses 120 and 130, an authentication server 11, and a network 12.

The authentication server 11 stores, in association with each other, identification information for identifying an individual and verification information (template) registered in advance prior to biometric authentication. The identification information is a unique identification assigned directly (e.g., a user number) or indirectly (e.g., a card number) to a user. The verification information is, for example, feature information extracted from image information in relation to features by a predetermined feature extraction algorithm, or encoded information generated by encoding the image information or the feature information.

The entrance and exit management apparatus 120 includes an authentication apparatus 121 and a door 125. The authentication apparatus 121 includes a numeric keypad 122, an IC card reader/writer 123, and a sensor unit 124. The numeric keypad 122 is used to enter a personal identification number in the case of concurrently using personal identification number authentication. The IC card reader/writer 123 reads and writes information from and to an IC card (not illustrated) of a user. The sensor unit 124 includes an image pickup device to capture an image of a palm of the user. The authentication apparatus 121 authenticates the user using a registered template stored in the IC card and the captured image and controls opening and closing of the door 125.

The sensor unit 124 is housed in an interior recess 124a. The interior recess 124a includes a built-in heater 124c. The heater 124c warms a space inside the interior recess 124a and a supporting part 124b for supporting a palm. When the user lays a hand over the sensor unit 124, the palm of the hand is warmed by warm air in the interior recess 124a and by the supporting part 124b. In this manner, the palm of the user is warmed, increasing blood flow in the palm.

The entrance and exit management apparatus 130 includes a flapper 132 and a sensor unit 131 for each gate. The sensor unit 131 includes an image pickup device to capture an image of a palm of the user. The entrance and exit management apparatus 130 authenticates the user using a registered template stored in the IC card and the captured image and controls opening and closing of the flapper 132.

The sensor unit 131 is provided on a height adjusting unit 133 for allowing the height of the sensor unit 131 to be adjusted. The sensor unit 131 is able to capture an image of a palm as changing the position in height of the palm. This changes the height of the palm of the user relative to his or her heart. Blood flow in the palm of the user reduces when the palm is at a higher position relative to the heart and increases when it is at a lower position. Therefore, the height adjusting unit 133 lowers the height position of the sensor unit 131 and, in this manner, functions as a posture changing unit for changing the posture of the user to thereby increase blood flow in the palm of the user.

Note that the entrance and exit management apparatus 130 may be equipped with multiple gates for each of which the sensor unit 131 is installed at a different height, and guide a user whose blood flow is desired to be increased to a gate with the sensor unit 131 installed at a lower height.

Alternatively, the entrance and exit management apparatus 130 may be equipped with a single gate on which multiple sensor units 131 are installed at different heights, and guide a user whose blood flow is desired to be increased to use the sensor unit 131 installed at a lower height.

### Fourth Embodiment

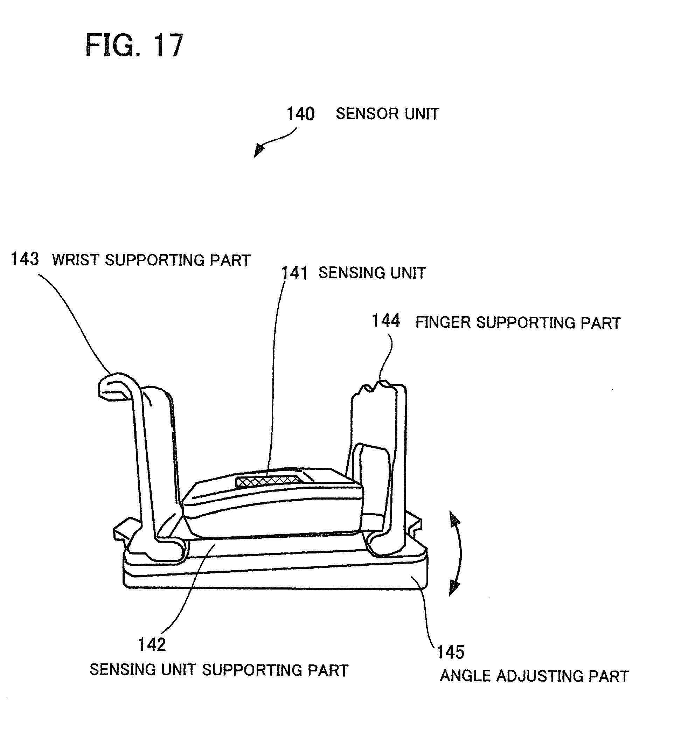

Next is described an overview of a sensor unit according to a fourth embodiment. FIG. 17 illustrates an overview of a sensor unit according to the fourth embodiment. The sensor unit of the fourth embodiment differs from that of the second embodiment in changing the angle of supporting a palm.

A sensor unit 140 includes a sensing unit 141, a sensing unit supporting part 142, a wrist supporting part 143, a finger supporting part 144, and an angle adjusting part 145. The sensing unit 141 includes a first image sensor for capturing an image of a living body part, a condenser lens, multiple near-infrared light emitting devices irradiating an object, and a second image sensor for capturing an image of temperature distribution of the living body part. The near-infrared light emitting devices emit near-infrared light in the direction of the object (i.e., the upper direction). The sensing unit supporting part 142 supports the sensing unit 141. The wrist supporting part 143 has a substantially U-shape configuration to support the wrist of the user in a correct posture. The finger supporting part 144 has a wave shape with two crests to support the index, third, and fourth fingers individually in their correct positions. The angle adjusting part 145 changes the tilt of the sensing unit supporting part 142, the wrist supporting part 143, and the finger supporting part 144. The angle adjusting part 145 changes the angle, for example, to -15, 0, and 15 degrees to thereby enable a palm of a user to be tilted backward, kept in a horizontal position, and tilted forward, respectively.

Note that the angle adjusting part 145 changes the posture of the palm by changing the tilt of the sensing unit supporting part 142, the wrist supporting part 143, and the finger supporting part 144. However, the angle adjusting part 145 may achieve the same effect by adjusting the heights of the wrist supporting part 143 and the finger supporting part 144 instead.

In this manner, the sensor unit 140 increases blood flow in the palm of a user by changing the posture of the palm. In addition, such a sensor unit 140 is highly suitable for use in a situation where the user takes a seated position. In the case of being used for template registration, the sensor unit 140 may position the palm of the user in a posture corresponding to a palm posture to be taken in an authentication process. For example, if a palm angle is to be 15 degrees during the image capturing process in an authentication apparatus used by the user, a palm angle for the template registration is also set to 15 degrees. In this case, the sensor unit 140 may additionally include a heater or other blood flow increasing means.

Note that the sensor unit 140 may increase blood flow in the palm of a user by repetition of operation for changing the palm posture or by a massage given by vibrating means such as an eccentric motor and an ultrasonic vibrator. Thus, the sensor unit 140 is able to increase blood flow in the palm of a user also by applying a stimulus thereto. Furthermore, the sensor unit 140 may additionally include a heater and achieve an increase in blood flow in a composite manner.

### Fifth Embodiment

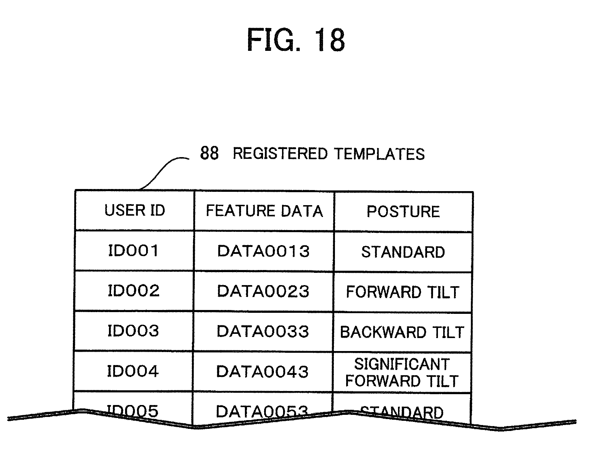

Next described are registered templates according to a fifth embodiment. FIG. 18 illustrates an example of registered templates according to a fifth embodiment. The registered templates of the fifth embodiment differ from those of the second embodiment in registering a posture taken when biometric information is acquired for each template registration.

A posture taken in each template registration is associated with both a user identification for uniquely identifying a user and a feature data set, and registered as a template as illustrated in registered templates 88. For example, the user with the user identification ID001 is associated with a feature data set DATA0013 and a posture "standard (horizontal)". The user with the user identification ID002 is associated with a feature data set DATA0023 and a posture "tilted forward". A user with a user identification ID003 is associated with a feature data set DATA0033 and a posture "tilted backward".

Thus, a posture taken in each template registration is also registered in a corresponding template, which allows the authentication apparatus to immediately select a posture enabling an increase in blood flow to acquire biometric information.

Note that an authentication apparatus (for example, the automated teller machine 30) may acquire surrounding environmental conditions causing changes in the amount of blood flow, such as seasons and temperature variations, and then cause the blood flow increasing unit 205 to operate if predetermined conditions representing a high likelihood of a reduction in blood flow having taken place are satisfied, for example, when the outside air temperature is equal to or less than a predetermined value.

Note that the blood flow increasing unit 205 is applicable not only for increasing blood flow but also reducing (i.e., increasing in the negative direction) blood flow. In this case, for example, the blood flow increasing unit 205 blows cool air to the palm of a user or positions the palm high. In this manner, the authentication apparatus is able to also match a feature amount of authentication biometric information to a feature amount of template-registered biometric information.

In addition, the above-described processing functions may be achieved by a computer. In this case, a program is provided in which processing contents of functions that each apparatus needs to have are described. By executing the program on the computer, the above-described processing functions are achieved on the computer. The program in which processing contents are described may be recorded in computer-readable recording media (including portable recording media). Such computer-readable recording media include a magnetic-storage device, an optical disk, a magneto-optical recording medium, and a semiconductor memory. Examples of the magnetic-storage device are a hard disk drive (HDD), a flexible disk (FD), and a magnetic tape. Examples of the optical disk are a digital versatile disk (DVD), a DVD random access memory (DVD-RAM), a compact disc read-only memory (CD-ROM), a CD recordable (CD-R), and a CD rewritable (CD-RW). An example of the magneto-optical recording medium is a magneto-optical disk (MO).

In the case of distributing the program, portable recording media, such as DVDs and CD-ROMs, in which the program is recorded are sold. In addition, the program may be stored in a memory device of a server computer and then transferred from the server computer to another computer via a network.

A computer for executing the program stores the program, which is originally recorded in a portable recording medium or transferred from the server computer, in its own memory device. Subsequently, the computer reads the program from its own memory device and performs processing according to the program. Note that the computer is able to read the program directly from the portable recording medium and perform processing according to the program. In addition, the computer is able to sequentially perform processing according to a received program each time such a program is transferred from the server computer.

Note that various modifications are possible to the above-described embodiments without departing from the scope of the embodiments.

The foregoing is merely illustrative of the principles of the present invention. Further, numerous modifications and changes will readily occur to those skilled in the art, and therefore, it is not desired to limit the disclosed technology to the exact construction and applications illustrated and described above. Accordingly, all suitable modifications and equivalents may be resorted to, falling within the scope of the present invention determined by appended claims and their equivalents.

### Reference Signs List

- 1: biometric information acquisition apparatus
- 2a: blood flow increasing unit
- 2b: biometric information acquiring unit
- 2c: feature amount evaluating unit
- 2d: reacquisition determining unit

## Claims

1. A biometric information acquisition apparatus comprising:
a blood flow increasing unit configured to increase an amount of blood flow of an object person;
a biometric information acquiring unit configured to acquire, from the object person, biometric information whose feature amount is to be increased due to an increase in the amount of blood flow;
a feature amount evaluating unit configured to evaluate the feature amount of the biometric information acquired by the biometric information acquiring unit; and
a reacquisition determining unit configured to determine whether to cause the blood flow increasing unit to operate and then cause the biometric information acquiring unit to reacquire the biometric information when the feature amount evaluated by the feature amount evaluating unit does not reach a predetermined threshold.

2. The biometric information acquisition apparatus according to claim 1, wherein the blood flow increasing unit includes a posture changing unit for changing a posture of a biometric information acquisition region from which the biometric information of the object person is acquired.

3. The biometric information acquisition apparatus according to claim 2, wherein the posture changing unit changes a tilt of a supporting part that supports the biometric information acquisition region.

4. The biometric information acquisition apparatus according to claim 2, wherein the posture changing unit changes height of a supporting part that supports the biometric information acquisition region.

5. The biometric information acquisition apparatus according to claim 2, wherein the posture changing unit changes a position of the biometric information acquisition region.

6. The biometric information acquisition apparatus according to claim 2, wherein the blood flow increasing unit includes a vibrating unit for applying a vibration to the biometric information acquisition region.

7. The biometric information acquisition apparatus according to claim 1,
wherein the biometric information acquiring unit includes an image capturing device for acquiring the biometric information, the image capturing device being disposed in an interior recess of the biometric information acquiring unit, and
the blood flow increasing unit includes a warming unit for warming inside the interior recess.

8. The biometric information acquisition apparatus according to claim 7,
wherein the reacquisition determining unit includes a temperature measuring unit for measuring temperature of the biometric information acquisition region, and
the reacquisition determining unit determines a timing of reacquiring the biometric information, based on a result of the temperature measurement of the biometric information acquisition region.

9. The biometric information acquisition apparatus according to claim 1, further comprising a verification information registering unit configured to register verification information based on the biometric information acquired by the biometric information acquiring unit.

10. The biometric information acquisition apparatus according to claim 9, wherein the verification information includes an evaluation made for the biometric information by the feature amount evaluating unit.

11. The biometric information acquisition apparatus according to claim 9, wherein the verification information includes information on an operation effected by the blood flow increasing unit.

12. The biometric information acquisition apparatus according to claim 9, wherein the blood flow increasing unit increases the amount of blood flow of the object person based on the verification information.

13. The biometric information acquisition apparatus according to claim 1, further comprising a verifying unit configured to verify the biometric information acquired by the biometric information acquiring unit against verification information registered in advance.

14. A biometric information acquisition method comprising:
acquiring, from an object person, biometric information whose feature amount is to be increased due to an increase in an amount of blood flow of the object person;
evaluating the feature amount of the acquired biometric information; and
increasing the amount of blood flow in a biometric information acquisition region from which the biometric information of the object person is acquired and reacquiring the biometric information from the biometric information acquisition region when the evaluated feature amount does not reach a predetermined threshold.
